# EUROPEAN PATENT SPECIFICATION

(11) **EP 1 828 108 B1**
(45) Date of publication and mention of the grant of the patent: **23.02.2011**
(21) Application number: 05815905.4
(22) Date of filing: 20.12.2005
(51) Int. Cl.: C07C 255/31

(54) **5-(2,2-DIMETHYL-CYCLOPRODYL)-3-METHYL-PEN-2-ENENITRILE AS FRAGRANCE AND FLAVOUR**
5-(2,2-DIMETHYL-CYCLOPROPYL)-3-METHYLPENT-2-ENNITRIL ALS GERUCHS- UND GESCHMACKSSTOFF
5-(2,2-DIMETHYL-CYCLOPRODYL)-3-METHYL-PEN-2-ENENITRILE ET SON UTILISATION EN PARFUMERIE ET AROMATISATION

(30) Priority: 24.12.2004 GB 0428306
(43) Date of publication of application: 05.09.2007
(73) Proprietor: Givaudan SA, 1214 Vernier-Genève (CH)
(72) Inventor: SCHRÖDER, Fridtjof, CH-8442 Hettlingen (CH)
(74) Representative: Givaudan Patents
(86) International application number: PCT/CH2005/000764
(87) International publication number: WO 2006/066437

(56) References cited:
- WO-A-03/053902

## Description

This invention relates to a novel compound.

The invention provides the compound 5-(2,2-dimethyl-cyclopropyl)-3-methyl-pent-2-enenitrile.

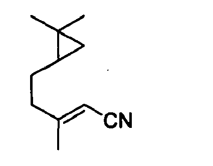

Compounds of this general type are known to the art, for example, International Patent Applications WO 03/053901 and WO 03/053902. However, nowhere in the art has this specific compound been identified, nor is its odour profile described.

The compound may be made by any convenient method, an especially preferred method being the cyclopropanation of a suitable substituted alkene, comprising the reaction of the alkene with a carbenoide, generated from dibromomethane and a tri-(C₂-C₈)-alkylaluminum compound.

The compound is useful as an ingredient in fragrance or flavour applications. The invention therefore also provides the use in a fragrance or flavour application of 5-(2,2-dimethylcyclopropyl)-3-methyl-pent-2-enenitrile. The fragrance application may be in any field of fine or functional fragrance, for example, perfumes, toiletries, cosmetics, soaps, laundry and household products and polishes. The flavour application may be in any kind of food product or flavouring.

In such applications, it may be combined with other known fragrance or flavour materials to provide desirable fragrance or flavour properties.

The invention is further described with reference to the following example.

### EXAMPLE

Cyclopropanation of *E*/*Z*-Geranitrile: *E*/*Z*-5-(2,2-dimethyl-cyclopropyl)-3-methyl-pent-2-enenitrile. 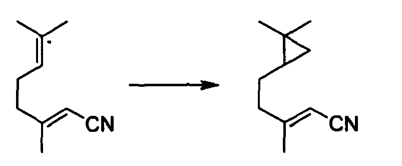 Anhydrous FeCl₃ (5 g, 30 mmol) is added under stirring and nitrogen to *E*/*Z*-Geranitrile (dr = 1:1) (77 g, 0.52 mol) in dibromomethane (720 ml, 10.3 mol) at 10° - 20°C, followed by slow addition of neat triisobutylaluminum (358 g, 1.8 mol) at this temperature. The mixture is stirred for 17 h at 25°C, then cooled to -10° - 0°C and pumped via double needle onto 25% NaOH at -10° - 0°C. Under stirring the biphasic mixture is slowly warmed up to room temperature. The organic phase is separated, dried over MgSO₄, filtered and the solvent is evaporated under reduced pressure.

The thus obtained oily residue is subjected to the same cyclopropanation cycle as above using anhydrous FeCl₃ (5 g, 30 mmol), dibromomethane (720 ml, 10.3 mol) and neat triisobutylaluminum (358 g, 1.8 mol). After 17 h at 25°C the mixture is cooled tao -10° - 0°C and pumped via double needle onto 25% NaOH at -10° - 0°C. Under stirring the biphasic mixture is slowly warmed up to room temperature. The organic phase is separated, washed with 4% oxalic acid, then with conc. NaHCO₃ until pH ~ 8, dried over MgSO₄ and filtered. After evaporation of the solvents under reduced pressure the oily residue is purified by distillation (bp 95°C /0,08 mbar (0.06 Torr)) giving 50 g (59%) of the cyclopropanation product (71% GC-purity, 8% aldehyde, 11% alcohol, dr = 1:1) as colorless oil. ¹H-NMR (CDCl₃, 400 MHz): 5.1 (1 H), 2.5 (m, 1 H), 2.25 (m, 1 H), 2.05 (s, 1.5 H), 1.9 (s, 1.5 H), 1.6 - 1.3 (2 H), 1.05 (6 H), 0.45 (2 H), -0.1 (1 H) ppm. ¹³C-NMR (CDCl₃, 400 MHz): 165.43 and 165.4 (2 s), 117.2 and 117.0 (2 s), 95.6 and 95.0 (2 d), 39.1 and 36.7 (2 t), 27.6 and 27.5 (2 t), 27.39 and 27.37 (2 q), 28.9 and 23.8 (2 d), 22.9 and 21.0 (2 q), 19.9 and 19.8 (2 q), 15.6 and 15.5 (2 s) ppm. GC/MS: 162 (20%), 148 (1%, [M-15]⁺), 94 (20%), 81 (55%), 55 (100%). IR (film): 2952 (s), 2867 (m), 2218 (w), 1676 (w), 1632 (w), 1454 (m), 1377 (m), 1365 (m), 1120 (w), 1020 (m), 866 (w), 801 (w). Odour: Hesperidic, powerful, fresh, geranitrile.

## Claims

1. 5-(2,2-dimethyl-cyclopropyl)-3-methyl-pent-2-enenitrile.

2. Use of 5-(2,2-dimethyl-cyclopropyl)-3-methyl-pent-2-enenitrile in a fragrance application.

3. A fragrance application comprising 5-(2,2-Dimethyl-cyclopropyl)-3-methyl-pent-2-enenitrile.

4. Use of 5-(2,2-Dimethyl-cyclopropyl)-3-methyl-pent-2-enenitrile in a flavour application.

5. A flavour application comprising 5-(2,2-dimethyl-cyclopropyl)-3-methyl-pent-2-enenitrile.

## Patentansprüche

1. 5-(2,2-Dimethylcyclopropyl)-3-methylpent-2-ennitril.

2. Verwendung von 5-(2,2-Dimethylcyclopropyl)-3-methylpent-2-ennitril bei einer Duftstoffanwendung.

3. Duftstoffanwendung, umfassend 5-(2,2-Dimethylcyclopropyl)-3-methylpent-2-ennitril.

4. Verwendung von 5-(2,2-Dimethylcyclopropyl)-3-methylpent-2-ennitril bei einer Geschmacksstoffanwendung.

5. Geschmacksstoffanwendung, umfassend 5-(2,2-Dimethylcyclopropyl)-3-methylpent-2-ennitril.

## Revendications

1. 5-(2,2-Diméthyl-cyclopropyl)-3-méthyl-pent-2-ènenitrile.

2. Utilisation du 5-(2,2-diméthyl-cyclopropyl)-3-méthyl-pent-2-ènenitrile dans une application de parfum.

3. Application de parfum comprenant le 5-(2,2-diméthyl-cyclopropyl)-3-méthyl-pent-2-ènenitrile.

4. Utilisation du 5-(2,2-diméthyl-cyclopropyl)-3-méthyl-pent-2-ènenitrile dans une application d'arôme.

5. Application d'arôme comprenant du 5-(2,2-diméthyl-cyclopropyl)-3-méthyl-pent-2-ènenitrile.
